# FASCICULE DE BREVET EUROPEEN

(11) **EP 3 124 025 B1**
(45) Date de publication et mention de la délivrance du brevet: **28.02.2018**
(21) Numéro de dépôt: 16181821.6
(22) Date de dépôt: 29.07.2016
(51) Int. Cl.: A61K 31/517, A61K 31/5377, A61P 35/00, A61K 45/06

(54) **ASSOCIATION ENTRE LE 3-[(3-{[4-(4-MORPHOLINYLMÉTHYL)-1H-PYRROL-2-YL]MÉTHYLÈNE}-2-OXO-2,3-DIHYDRO-1H-INDOL-5-YL)MÉTHYL]-1,3-THIAZOLIDINE-2,4-DIONE ET UN INHIBITEUR DE LA TYR KINASE DU EGFR**
ASSOZIATION ZWISCHEN 3-[(3-{[4-(4-MORPHOLINYLMETHYL)-1H-PYRROL-2-YL]METHYLEN}-2-OXO-2,3-DIHYDRO-1H-INDOL-5-YL)METHYL]-1,3-THIAZOLIDIN-2,4-DION UND EINEM TYROSIN-KINASEHEMMER DES EGF-REZEPTORS
COMBINATION BETWEEN 3-[(3-{[4-(4-MORPHOLINYLMETHYL)-1H-PYRROL-2-YL] METHYLENE}-2-OXO-2,3-DIHYDRO-1H-INDOL-5-YL)METHYL]-1,3-THIAZOLIDINE-2,4-DIONE AND AN EGFR TYR-KINASE INHIBITOR

(30) Priorité: 31.07.2015 FR 1557412
(43) Date de publication de la demande: 01.02.2017
(73) Titulaire: Les Laboratoires Servier, 92284 Suresnes Cedex (FR)
(72) Inventeur: BURBRIDGE, Michaël, Frank, 78480 VERNEUIL SUR SEINE (FR); CATTAN, Valérie, 75007 PARIS (FR); JACQUET-BESCOND, Anne, 92350 LE PLESSIS ROBINSON (FR)

(56) Documents cités:
- WO-A1-2011/015728
- WO-A1-2015/004395
- WO-A1-2016/055916
- WO-A2-2014/138364
- M. F. BURBRIDGE ET AL: "S49076 Is a Novel Kinase Inhibitor of MET, AXL, and FGFR with Strong Preclinical Activity Alone and in Association with Bevacizumab", MOLECULAR CANCER THERAPEUTICS, vol. 12, no. 9, 26 juin 2013 (2013-06-26), pages 1749-1762, XP055113861, ISSN: 1535-7163, DOI: 10.1158/1535-7163.MCT-13-0075
- XIAOLIANG WU ET AL: "AXL kinase as a novel target for cancer therapy Introduction to the TAM tyrosine kinase receptor family", ONCOTARGET, vol. 5, 16 octobre 2014 (2014-10-16), XP055242570,
- J. K. RHO ET AL: "MET and AXL Inhibitor NPS-1034 Exerts Efficacy against Lung Cancer Cells Resistant to EGFR Kinase Inhibitors Because of MET or AXL Activation", CANCER RESEARCH, vol. 74, no. 1, 28 octobre 2013 (2013-10-28), pages 253-262, XP055273129, US ISSN: 0008-5472, DOI: 10.1158/0008-5472.CAN-13-1103
- YUN JUNG CHOI ET AL: "AUY922 Effectively Overcomes MET- and AXL-Mediated Resistance to EGFR-TKI in Lung Cancer Cells", PLOS ONE, vol. 10, no. 3, 17 mars 2015 (2015-03-17), page e0119832, XP055273140, DOI: 10.1371/journal.pone.0119832
- PÉREZ-RAMÍREZ CRISTINA ET AL: "MET/HGF targeted drugs as potential therapeutic strategies in non-small cell lung cancer", PHARMACOLOGICAL RESEARCH, vol. 102, 28 septembre 2015 (2015-09-28), pages 90-106, XP029339654, ISSN: 1043-6618, DOI: 10.1016/J.PHRS.2015.09.016

## Description

La présente invention concerne l'association entre le 3-[(3-{[4-(4-morpholinylméthyl)-1*H*-pyrrol-2-yl]méthylène}-2-oxo-2,3-dihydro-1*H*-indol-5-yl)méthyl] -1,3-thiazolidine-2,4-dione de formule (I) : 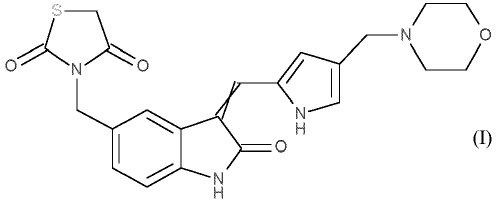 ou un de ses isomères Z ou E et/ou sels d'addition à un acide ou à une base pharmaceutiquement acceptable, et un inhibiteur de tyrosine kinase du récepteur du facteur de croissance épidermique humain (Epidermal Growth Factor Receptor, EGFR), ainsi que son utilisation pour le traitement du cancer du poumon non à petites cellules, et plus particulièrement chez les patients résistants à un inhibiteur de tyrosine kinase du EGFR.
Le cancer du poumon non à petites cellules est aujourd'hui la première cause de décès par cancer dans le monde (Goldstraw, P., D. Ball, J. R. Jett, C. T. Le, E. Lim, A. G. Nicholson, and F. A. Shepherd, 2011, Non-small-cell lung cancer: Lancet, v. 378, no. 9804, p. 1727-1740; Jemal, A., F. Bray, M. M. Center, J. Ferlay, E. Ward, and D. Forman, 2011, Global cancer statistics: CA Cancer J Clin, v. 61, no. 2, p. 69-90). Au moment du diagnostic, la majorité des patients ont une pathologie avancée avec une survie à un an de 30% et une survie à 5 ans de 10% (U.S. National Institutes of Health, National Cancer Institute http://seer.cancer.gov/archive/csr/1975_2011/results_merged/topic_delaygraphs_overview. pdf; http://www.cancerresearchuk.org/cancer-info/cancerstats/types/lung/survival/lung-cancer-survival-statistics). Les mutations activatrices du gène EGFR entrainent une addiction oncogénique c'est à dire la dépendance de la cellule cancéreuse à cette anomalie pour sa croissance et sa survie. Ces mutations sont fréquentes dans l'adénocarcinome du poumon avec 15% des cas chez les patients caucasiens et 40-50% des cas chez les patients asiatiques (Shigematsu, H. et al., 2005, Clinical and biological features associated with epidermal growth factor receptor gene mutations in lung cancers: J Natl Cancer Inst, v. 97, no. 5, p. 339-346). Chez les patients présentant une mutation du gène EGFR, les inhibiteurs de tyrosine kinase du EGFR retardent significativement la progression de la maladie comparée à la chimiothérapie et sont considérés comme traitement de référence. Les traitements actuels sur le marché sont entre autres le Géfitinib et l'Erlotinib pour les inhibiteurs de première génération, et l'Afatinib pour les inhibiteurs de seconde génération, chaque génération ciblant des mutations actives de EGFR. Malheureusement, la plupart des patients rechutent après plusieurs mois de traitement par l'acquisition d'autres altérations génétiques et protéiques capables d'engendrer une résistance aux inhibiteurs de tyrosine kinase du EGFR. Plusieurs mécanismes de résistance ont été identifiés, et notamment une nouvelle mutation du récepteur EGFR (T790M: substitution en position 790 d'une Thréonine par une Méthionine) est majoritairement retrouvée chez les patients résistant aux traitements. Après résistance aux inhibiteurs de tyrosine kinase du EGFR, le pronostic devient très faible et les patients se voient proposer une chimiothérapie ayant un faible taux d'efficacité. Dans ce contexte, la recherche de nouvelles alternatives thérapeutiques dans le cancer du poumon non à petites cellules, et particulièrement chez les patients résistants aux inhibiteurs de tyrosine kinase du EGFR, en vue d'améliorer la survie sans progression, reste toujours d'actualité. En particulier, resensibiliser les patients résistants aux inhibiteurs de tyrosine kinase du EGFR constitue une stratégie thérapeutique forte à explorer. Des inhibiteurs de tyrosine kinase du EGFR de troisième génération sont en cours de développement pour agir spécifiquement chez les patients ayant acquis une mutation secondaire comme la T790M par exemple, et semblent restaurer l'activité du traitement. D'autres alternatives, agissant sur d'autres voies de résistance que la mutation T790M et/ou impliquant d'autres récepteurs cellulaires sont toujours nécessaires et très attendues par les patients, et pourront notamment être associées avec des inhibiteurs de troisième génération. X. Wu et al. divulguent dans Oncotarget, 5(20), 9546, 2014, que la sensibilité de cancers après une résistance peut être restaurée en ciblant AXL. Le SGI17079, un inhibiteur de AXL, en association avec l'Erlotinib, renverse la résistance à l'Erlotinib dans un cancer du poumon non à petites cellules. Rho et al. divulguent dans Cancer Research, 74(1), 253, 2013, que l'association du Géfitinib ou du Erlotinib avec le NPS-1034 inhibe la prolifération cellulaire et induit la mort cellulaire des cellules cancéreuses de cancer du poumon non à petites cellules résistantes au Géfitinib ou au Erlotinib de façon synergique. Le NPS-1034 cible à la fois MET et AXL. WO-A-2014/138364 divulgue une combinaison d'un inhibiteur de FGFR et d'un inhibiteur de tyrosine kinase du EGFR pour le traitement du cancer du poumon non à petites cellules chez des patients ayant des mutations EGFR. Dans tout ce qui suit et lorsque rien d'autre n'est précisé, par "3-[(3-{[4-(4-morpholinyl-méthyl)-1*H*-pyrrol-2-yl]méthylène}-2-oxo-2,3-dihydro-1*H*-indol-5-yl)méthyl]-1,3-thiazolidine-2,4-dione" on entend "3-[(3- {[4-(4-morpholinyl-méthyl)-1*H*-pyrrol-2-yl] méthylène}-2-oxo-2,3-dihydro-1*H*-indol-5-yl)méthyl]-1,3-thiazolidine-2,4-dione, ainsi que ses isomères Z ou E et/ou sels d'addition à un acide ou à une base pharmaceutiquement acceptable ».

Le 3-[(3-{[4-(4-morpholinylméthyl)-1*H*-pyrrol-2-yl]méthylène}-2-oxo-2,3-dihydro-1*H-*indol-5-yl)méthyl]-1,3-thiazolidine-2,4-dione, est un puissant inhibiteur de la migration des cellules cancéreuses particulièrement utile pour le traitement des cancers et notamment des tumeurs solides métastatiques. Il est décrit dans les demandes de brevet WO2011/015728 et WO2015/004395.

Selon l'invention, il a été montré que les effets du 3-{[(3-{[4-(4-morpholinylmethyl)-1*H-*pyrrol-2-yl]methylene}-2-oxo-2,3-dihydro-1*H*-indol-5-yl]methyl}-1 ,3-thiazolidine-2,4-dione permettaient de lever la résistance aux inhibiteurs de tyrosine kinase du EGFR dans des modèles animaux préalablement traités par un tel inhibiteur.

Ces effets permettent d'envisager l'utilisation de l'association du 3-{[(3-{[4-(4-morpholinylmethyl)-1*H*-pyrrol-2-yl]methylene}-2-oxo-2,3-dihydro-1*H*-indol-5-yl]methyl} -1,3-thiazolidine-2,4-dione, et d'un inhibiteur de tyrosine kinase du EGFR dans le traitement des cancers du poumon non à petites cellules, et notamment chez des patients pour lesquels une progression de la maladie ou une rechute a été observée malgré le traitement.

Plus particulièrement dans l'association selon l'invention le 3-[(3-{[4-(4-morpholinyl-méthyl)-1*H*-pyrrol-2-yl]méthylène}-2-oxo-2,3-dihydro-1*H*-indol-5-yl)méthyl]-1,3-thiazolidine-2,4-dione est sous la forme de l'isomère Z.

Préfrentiellement dans l'association selon l'invention le 3-[(3- {[4-(4-morpholinyl-méthyl)-1*H*-pyrrol-2-yl]méthylène}-2-oxo-2,3-dihydro-1*H*-indol-5-yl)méthyl]-1,3-thiazolidine-2,4-dione est sous la forme d'un sel, et notamment un chlorhydrate ou un mésylate.

Encore plus avantageusement l'association selon l'invention contient le 3-[((3Z)-3-{[4-(4-morpholinyl-méthyl)-1*H*-pyrrol-2-yl]méthylène}-2-oxo-2,3-dihydro-1*H*-indol-5-yl) méthyl]-1,3-thiazolidine-2,4-dione mésylate.

Parmi les inhibiteurs de tyrosine kinase du EGFR selon l'invention, on peut citer l'Erlotinib, le Géfitinib et l'Afatinib pour les inhibiteurs de première et seconde génération, et l'AZD9291(Osimertinib) ou le Rociletinib pour les inhibiteurs de troisième génération.

Selon un mode de réalisation avantageux, l'inhibiteur de tyrosine kinase du EGFR de l'association selon l'invention est le N-(3-éthynylphényl)-6,7-di(2-méthoxyéthoxy) quinazolin-4-amine ou Erlotinib de formule (II) : 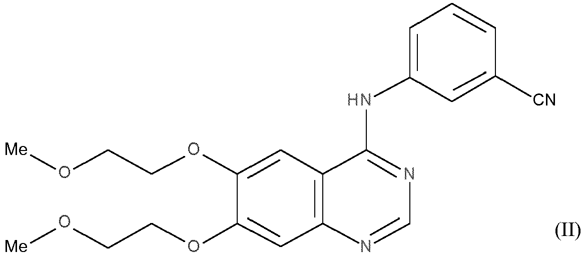 ou un de ses sels d'addition à un acide ou à une base pharmaceutiquement acceptable, et notamment son chlorhydrate.

Selon un autre mode de réalisation avantageux, l'inhibiteur de tyrosine kinase du EGFR de l'association selon l'invention est le *N*-(3-chloro-4-fluoro-phényl)-7-méthoxy-6-(3-morpholin-4-ylpropoxy)quinazolin-4-amine ou Géfitinib de formule (III) : 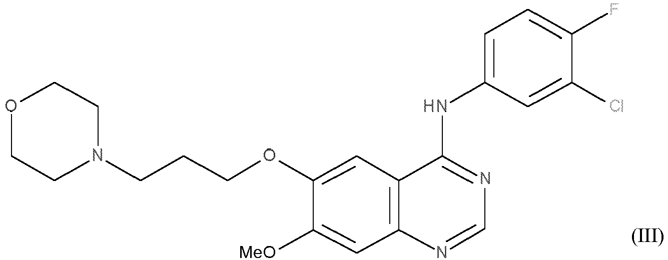 ou un de ses sels d'addition à un acide ou à une base pharmaceutiquement acceptable.
De manière préférentielle, l'association selon l'invention concerne l'isomère Z du 3-[(3-{[4-(4-morpholinyl-méthy1)-1*H*-pyrrol-2-yl]méthylène}-2-oxo-2,3-dihydro-1*H*-indol-5-yl)méthyl]-1,3-thiazolidine-2,4-dione ou un de ses sels pharmaceutiquement acceptable, avec le Géfitinib ou un de ses sels pharmaceutiquement acceptable.

Encore plus particulièrement, l'invention concerne l'association entre le 3-[((3Z)-3-{[4-(4-morpholinyl-méthyl)-1*H*- pyrrol-2-yl]méthylène}-2-oxo-2,3-dihydro-1*H*-indol-5-yl) méthyl]-1,3-thiazolidine-2,4-dione mésylate, avec le Géfitinib ou un de ses sels pharmaceutiquement acceptable.

L'invention concerne également les compositions pharmaceutiques contenant l'association entre le 3-[(3- {[4-(4-morpholinyl-méthyl)-1*H*-pyrrol-2-yl]méthylène }-2-oxo-2,3-dihydro-1*H*-indol-5-yl)méthyl]-1,3-thiazolidine-2,4-dione, et un inhibiteur de tyrosine kinase du EGFR en combinaison avec un ou plusieurs excipients pharmaceutiquement acceptables.

L'invention concerne aussi lesdites compositions pharmaceutiques pour leur utilisation dans le traitement du cancer du poumon non à petites cellules, et plus particulièrement chez les patients résistants à un inhibiteur de tyrosine kinase du EGFR.

Parmi les compositions pharmaceutiques selon l'invention, on retiendra plus particulièrement celles qui conviennent pour l'administration par voie orale, parentérale, intramusculaire et intraveineuse, per ou transcutanée, nasale, rectale, perlinguale, oculaire, respiratoire et plus spécifiquement les comprimés simples ou dragéifiés, les comprimés sublinguaux, les gélules, les glossettes, les capsules, les tablettes, les préparations injectables, les aérosols, les gouttes oculaires ou nasales, les suppositoires, les crèmes, pommades, gels dermiques, etc...

Dans un mode de réalisation préféré, le 3-[(3-{[4-(4-morpholinyl-méthyl)-1*H*-pyrrol-2-yl]méthylène}-2-oxo-2,3-dihydro-1*H*-indol-5-yl)méthyl]-1,3-thiazolidine-2,4-dione est administré sous forme orale.

Outre le 3-[(3- {[4-(4-morpholinyl-méthyl)-1*H*-pyrrol-2-yl]méthylène }-2-oxo-2,3-dihydro-1*H*-indol-5-yl)méthyl]-1,3-thiazolidine-2,4-dione et l'inhibiteur de tyrosine kinase du EGFR, les compositions pharmaceutiques selon l'invention contiennent un ou plusieurs excipients ou véhicules choisis parmi des diluants, des lubrifiants, des liants, des agents de désintégration, les stabilisants, les conservateurs, des absorbants, des colorants, des édulcorants, les aromatisants, etc...

A titre d'exemple, on peut citer:
- pour les diluants : le lactose, le dextrose, le sucrose, le mannitol, le sorbitol, la cellulose, la glycérine ;
- pour les lubrifiants : la silice, le talc, l'acide stéarique et ses sels de magnésium et de calcium, le polyéthylène glycol ;
- pour les liants : le silicate d'aluminium et de magnésium, l'amidon, la gélatine, la tragacanthe, la méthylcellulose, la carboxyméthylcellulose de sodium et la polyvinylpyrrolidone ;
- pour les désintégrants : l'agar, l'acide alginique et son sel de sodium, les mélanges effervescents.

Les composés de l'association peuvent être administrés de manière simultanée ou séquentielle. Les compositions pharmaceutiques correspondantes peuvent permettre la libération instantanée ou différée des principes actifs. Par ailleurs, les composés de l'association peuvent être administrés sous la forme de deux compositions pharmaceutiques distinctes, contenant chacune l'un des principes actifs, ou bien sous la forme d'une seule composition pharmaceutique, dans laquelle les principes actifs sont mélangés.

La posologie utile varie selon le sexe, l'âge et le poids du patient, la voie d'administration, la nature du cancer et des traitements éventuellement associés, et s'échelonne entre 300 et 1500 mg équivalents base de 3-[(3-{[4-(4-morpholinyl-méthyl)-1H-pyrrol-2-yl]méthylène} -2-oxo-2,3-dihydro-1H-indol-5-yl)méthyl]-1,3-thiazolidine-2,4-dione par jour, et plus préférentiellement entre 400 et 800 mg équivalents base par jour, et encore plus particulièrement entre 500 et 600 mg équivalents base par jour. La dose de l'inhibiteur de tyrosine kinase du EGFR sera égale à celle utilisée lorsqu'il est administré seul ou inférieure. A titre d'exemple, dans le cas du Géfitinib, la dose administrée est de 250 mg par jour. Pour l'Erlotinib, elle est de 25 à 150 mg par jour.

**COMPOSITION PHARMACEUTIQUE**

| | |
|---|---|
| 1000 comprimés dosés à 100 mg équivalent base du 3-[3-{[4-(4-morpholinylméthyl)-1*H*-pyrrol-2-yl] méthylène}-2-oxo-2,3-dihydro-1*H*-indol-5-yl)méthyl]-1,3-thiazolidine-2,4-dione méthanesulfonate, isomère Z | 121 g |
| Glycolate sodique d'amidon | 20 g |
| Amidon de maïs | 133 g |
| Lactose monohydrate | 357 g |
| Stéarate de magnésium | 6,7 g |
| Silice | 1,3 g |
| Povidone | 46,6 g |

### ETUDES PRECLINIOUES

### A) Test de viabilité cellulaire sur la lignée cellulaire HCC827 résistante à l'Erlotinib

Un test de viabilité cellulaire permettant de mesurer le pouvoir antiprolifératif des composés anti-tumoraux a été utilisé. La lignée cellulaire choisie est la lignée HCC827, une lignée de cancer du poumon non à petites cellules dépendante du EGFR pour sa survie. Le paramètre retenu est l'IC50, soit la concentration de produit inhibant 50% de la prolifération cellulaire par comparaison aux cellules témoins non traitées. Les cellules sont ensemencées (150µl) à la densité adéquate 2 jours avant l'expérience dans les puits de plaques 96 puits. Une colonne contient les cellules témoins non traitées représentant 100% de prolifération. Les autres sont incubés avec les produits à tester sur 4 temps de dédoublement. La concentration inhibitrice médiane de l'inhibiteur de tyrosine kinase du EGFR Erlotinib pour la viabilité cellulaire de la lignée HCC827 est de 10 nM. Une résistance acquise à l'Erlotinib est générée par exposition chronique de la lignée HCC827 à l'Erlotinib : les cellules sont exposées à l'Erlotinib à la dose de 1 µM dans le milieu de culture jusqu'à stabilisation du temps de dédoublement, soit environ 2 mois. La concentration inhibitrice médiane de l'Erlotinib pour la viabilité cellulaire de la lignée HCC827 résistante est alors environ 1000 fois plus élevée à 11,5 µM. Les cellules résistantes sont alors exposées au 3-[(3-{[4-(4-morpholinyl-méthyl)-1H-pyrrol-2-yl]méthylène}-2-oxo-2,3-dihydro-1H-indol-5-yl)méthyl]-1,3-thiazolidine-2,4-dione, chlorhydrate à une concentration de 100 nM en combinaison avec des doses croissantes d'Erlotinib. Le 3-[(3- {[4-(4-morpholinyl-méthyl)-1H-pyrrol-2-yl]méthylène}-2-oxo-2,3-dihydro-1H-indol-5-yl)méthyl]-1,3-thiazolidine-2,4-dione chlorhydrate, seul, n'a pas d'effet sur la viabilité. En combinaison, la concentration inhibitrice médiane de l'Erlotinib sur la lignée HCC827 résistante redevient de l'ordre de celle de la lignée HCC827 non résistante à 3,8 nM.

Ce résultat montre que le 3-[(3-{[4-(4-morpholinyl-méthyl)-1H-pyrrol-2-yl]méthylène}-2-oxo-2,3-dihydro-1H-indol-5-yl)méthyl]-1,3-thiazolidine-2,4-dione est capable de restaurer la sensibilité à un inhibiteur de tyrosine kinase du EGFR dans une lignée cellulaire de cancer du poumon non à petites cellules résistante à un tel inhibiteur.

### B) Inhibition de la croissance de la tumeur HCC827 résistante à l'Erlotinib

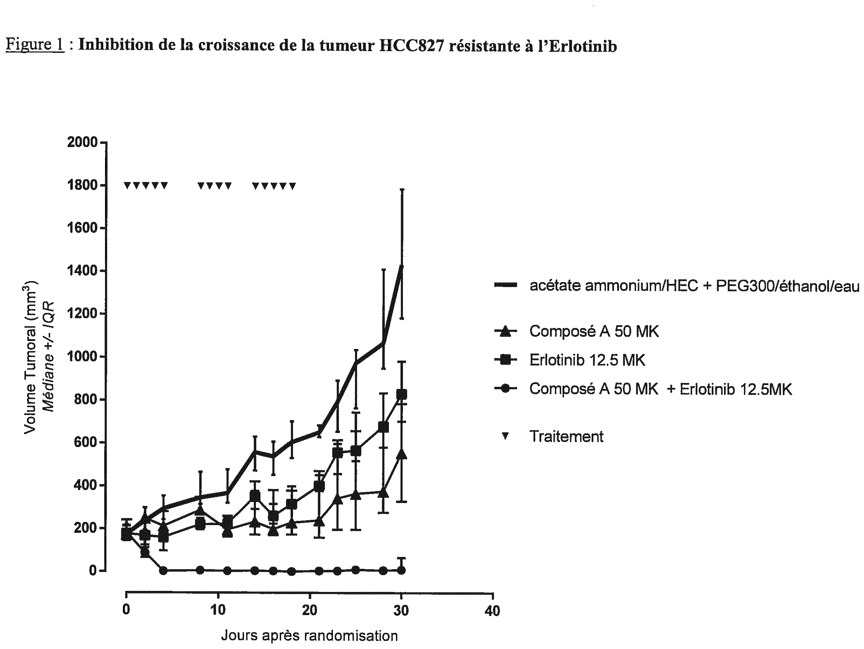

La lignée HCC827, lignée de cancer du poumon à non petites cellules, rendue résistante *in vitro* à l'Erlotinib, a été greffée en localisation sous-cutanée sur la souris SCID femelle à raison de 5.10⁶ cellules par souris. Les tumeurs ont été randomisées en groupes de huit souris lorsque le volume tumoral a atteint environ 200 mm³. Les traitements quotidiens pour le composé A (3-[(3-{[4-(4-morpholinyl-méthyl)-1H-pyrrol-2-yl]méthylène}-2-oxo-2,3-dihydro-1H-indol-5-yl)méthyl]-1,3-thiazolidine-2,4-dione, chlorhydrate) à la dose de 50 mg/kg, et pour l'Erlotinib à la dose de 12,5 mg/kg, ont été administrés par voie orale (véhicules = tampons acétate d'ammonium/HEC et PEG300/Ethanol/eau respectivement) sur une période de 19 jours comme indiqué par les triangles sur la figure 1. Les volumes tumoraux ont été mesurés à raison de deux à trois fois par semaine au pied à coulisse. Les volumes tumoraux médians avec les écarts interquartiles sont reportés sur le graphe.

A la fin des traitements, à J19, l'inhibition de la croissance après traitement par les composés seuls est de 65% pour l'Erlotinib et de 83 % pour le 3-[(3-{[4-(4-morpholinyl-méthyl)-1H-pyrrol-2-yl]méthylène}-2-oxo-2,3-dihydro-1H-indo1-5-yl)méthyl]-1,3-thiazolidine-2,4-dione, chlorhydrate (Composé A). Lorsque les 2 composés sont utilisés en combinaison, les tumeurs régressent complètement, et cette régression persiste dans le temps, après l'arrêt des traitements jusqu'à J30. La synergie observée entre les 2 produits est statistiquement significative sur le temps de l'étude (p<0,001).

Les résultats sont reportés dans la Figure 1.

### ETUDE CLINIQUE

Les patients ayant un cancer du poumon non à petites cellules développent une résistance au traitement par un inhibiteur de tyrosine kinase du EGFR (Gefitinib, Erlotinib, Afatinib, Osimertinib ou Rociletinib), et ne sont plus sensibles au traitement et la maladie progresse. Une étude de preuve de concept est en cours afin de confirmer les résultats observés dans les études précliniques et de montrer que le 3-[(3-{[4-(4-morpholinyl-méthyl)-1H-pyrrol-2-yl]méthylène}-2-oxo-2,3-dihydro-1H-indol-5-yl)méthyl]-1,3-thiazolidine-2,4-dione est capable de restaurer la sensibilité à un inhibiteur d'EGFR, en l'occurrence le Géfitinib chez des patients atteints d'un cancer du poumon non à petites cellules devenus résistants. Les patients sont inclus selon leur profil moléculaire. Cette étude comporte une phase I ayant pour objectif d'évaluer le profil de tolérance et de déterminer la dose recommandée pour la suite du développement. Environ 20 patients seront inclus. Pendant cette phase les patients seront traités par cycles de 28 jours avec une dose de 400, 500 ou 600 mg par jour d'équivalent base du 3-[(3-{[4-(4-morpholinyl-méthyl)-1H-pyrrol-2-yl]méthylène}-2-oxo-2,3-dihydro-1H-indol-5-yl) méthyl]-1,3-thiazolidine-2,4-dione en combinaison avec 250 mg par jour de Géfitinib. Le traitement sera maintenu jusqu'à progression de la maladie. A l'issue de cette phase, une phase II sera initiée ayant pour objectif d'évaluer l'activité de la combinaison entre le 3-[(3-{[4-(4-morpholinyl-méthyl)-1H-pyrrol-2-yl]méthylène}-2-oxo-2,3-dihydro-1H-indol-5-yl)méthyl]-1,3-thiazolidine-2,4-dione et le Géfitinib. Cette phase II incluera environ 150 patients. L'évaluation tumorale sera réalisée tous les 2 mois. Les patients seront traités par cycles de 28 jours avec la dose recommandée de 3-[(3-{[4-(4-morpholinyl-méthyl)-1H-pyrrol-2-yl]méthylène } -2-oxo-2,3-dihydro-1H-indol-5-yl) méthyl]-1,3-thiazolidine-2,4-dione définie dans la phase I en combinaison avec 250 mg de Géfitinib par jour.

## Revendications

1. Association entre le 3-[(3-{[4-(4-morpholinylméthyl)-1*H*-pyrrol-2-yl]méthylène}-2-oxo-2,3-dihydro-1*H*-indol-5-yl)méthyl]-1,3-thiazolidine-2,4-dione de formule (I) : 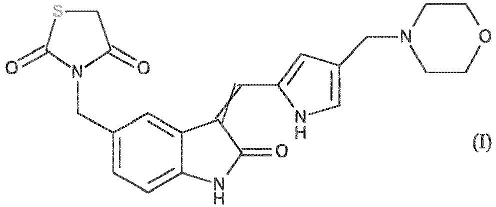 ainsi que ses isomères Z ou E et/ou sels d'addition à un acide ou à une base pharmaceutiquement acceptable, et un inhibiteur de tyrosine kinase du récepteur du facteur de croissance épidermique humain (EGFR).

2. Association selon la revendication 1 **caractérisée en ce que** le 3-[(3-{[4-(4-morpholinylméthyl)-1*H*-pyrrol-2-yl]méthylène}-2-oxo-2,3-dihydro-1*H*-indol-5-yl)méthyl] -1,3-thiazolidine-2,4-dione est utilisé sous la forme de l'isomère Z.

3. Association selon l'une des revendications 1 ou 2 **caractérisée en ce que** le 3-[(3-{[4-(4-morpholinylméthyl)-1*H*-pyrrol-2-yl]méthylène}-2-oxo-2,3-dihydro-1*H*-indol-5-yl)méthyl] -1,3-thiazolidine-2,4-dione est utilisé sous la forme d'un chlorhydrate.

4. Association selon l'une des revendications 1 ou 2 **caractérisée en ce que** le 3-[(3-{[4-(4-morpholinylméthyl)-1*H*-pyrrol-2-yl]méthylène}-2-oxo-2,3-dihydro-1*H*-indol-5-yl)méthyl] -1,3-thiazolidine-2,4-dione est utilisé sous la forme d'un mésylate.

5. Association selon l'une des revendications 1 à 4 **caractérisée en ce que** l'inhibiteur de Tyrosine kinase du EGFR est le Géfitinib ou l'Erlotinib.

6. Association selon l'une des revendications 1 à 5 pour son utilisation dans le traitement du cancer du poumon non à petites cellules.

7. Association selon l'une des revendications 1 à 5 pour son utilisation dans le traitement du cancer du poumon non à petites cellules chez les patients résistants à un inhibiteur de Tyrosine kinase du EGFR.

8. Composition pharmaceutique contenant comme principe actif le 3-[(3-{[4-(4-morpholinylméthyl)-1*H*-pyrrol-2-yl]méthylène}-2-oxo-2,3-dihydro-1*H*-indol-5-yl)méthyl] -1,3-thiazolidine-2,4-dione ou un de ses isomères Z ou E et/ou sels d'addition à un acide ou à une base pharmaceutiquement acceptable, en association avec un inhibiteur de la Tyrosine kinase du EGFR selon l'une des revendications 1 à 7 en combinaison avec un ou plusieurs excipients pharmaceutiquement acceptables.

9. Composition pharmaceutique selon la revendication 8 pour son utilisation dans le traitement du cancer du poumon non à petites cellules.

10. Composition pharmaceutique selon la revendication 9 pour son utilisation dans le traitement du cancer du poumon non à petites cellules chez les patients résistants à un inhibiteur de Tyrosine kinase du EGFR.

## Patentansprüche

1. Kombination aus 3-[(3-{[4-(4-Morpholinylmethyl)-1*H*-pyrrol-2-yl]-methylen}-2-oxo-2,3-dihydro-1*H*-indol-5-yl)-methyl]-1,3-thiazolidin-2,4-dion der Formel (I): 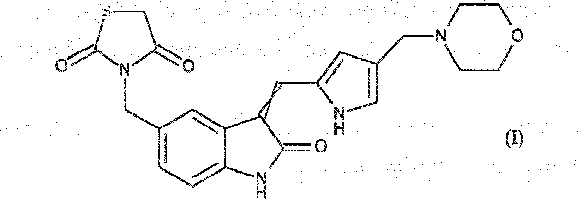 sowie dessen Z- oder E-Isomeren und/oder Additionssalze mit einer pharmazeutisch annehmbaren Säure oder Base und einem Inhibitor der Tyrosinkinase des Rezeptors des menschlichen epidermalen Wachstumsfaktors (EGFR).

2. Kombination nach Anspruch 1, **dadurch gekennzeichnet, dass** 3-[(3-{[4-(4-Morpholinylmethyl)-1*H*-pyrrol-2-yl]-methylen}-2-oxo-2,3-dihydro-1*H*-indol-5-yl)-methyl]-1,3-thiazolidin-2,3-dion in Form des Z-Isomeren verwendet wird.

3. Kombination nach einem der Ansprüche 1 oder 2, **dadurch gekennzeichnet, dass** das 3-[(3-{[4-(4-Morpholinylmethyl)-1*H*-pyrrol-2-yl]-methylen}-2-oxo-2,3-dihydro-1*H*-indol-5-yl)-methyl]-1,3-thiazolidin-2,3-dion in Form eines Hydrochlorids verwendet wird.

4. Kombination nach einem der Ansprüche 1 oder 2, **dadurch gekennzeichnet, dass** das 3-[(3-{[4-(4-Morpholinylmethyl)-1*H*-pyrrol-2-yl]-methylen}-2-oxo-2,3-dihydro-1*H*-indol-5-yl)-methyl]-1,3-thiazolidin-2,3-dion in Form eines Mesylats verwendet wird.

5. Kombination nach einem der Ansprüche 1 bis 4, **dadurch gekennzeichnet, dass** der Inhibitor der Tyrosinkinase von EGFR Gefitinib oder Erlotinib ist.

6. Kombination nach einem der Ansprüche 1 bis 5 zur Verwendung bei der Behandlung von nicht-kleinzelligem Lungenkrebs.

7. Kombination nach einem der Ansprüche 1 bis 5 zur Verwendung bei der Behandlung von nicht-kleinzelligem Lungenkrebs bei Patienten, die gegen einen Inhibitor der Tyrosinkinase von EGFR resistent sind.

8. Pharmazeutische Zubereitung enthaltend als Wirkstoff 3-[(3-{[4-(4-Morpholinylmethyl)-1*H*-pyrrol-2-yl]-methylen}-2-oxo-2,3-dihydro-1*H*-indol-5-yl)-methyl]-1,3-thiazolidin-2,3-dion oder eines seiner Z- oder E-Isomeren und/oder Additionssalze mit einer pharmazeutisch annehmbaren Säure oder Base in Kombination mit einem Inhibitor der Tyrosinkinase von EGFR nach einem der Ansprüche 1 bis 7 in Kombination mit einem oder mehreren pharmazeutisch annehmbaren Hilfsstoffen.

9. Pharmazeutische Zubereitung nach Anspruch 8 zur Verwendung bei der Behandlung von nicht-kleinzelligem Lungenkrebs.

10. Pharmazeutische Zubereitung nach Anspruch 9 zur Verwendung bei der Behandlung von nicht-kleinzelligem Lungenkrebs bei Patienten, die gegen einen Inhibitor der Tyrosinkinase von EGFR resistent sind.

## Claims

1. Association between 3-[(3-{[4-(4-morpholinylmethyl)-1*H*-pyrrol-2-yl]methylene}-2-oxo-2,3-dihydro-1*H*-indol-5-yl)methyl]-1,3-thiazolidine-2,4-dione of formula (I): 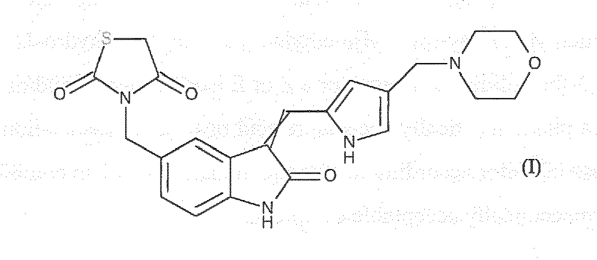 and its Z or E isomers and/or addition salts with a pharmaceutically acceptable acid or base, and a human epidermal growth factor receptor (EGFR) tyrosine kinase inhibitor.

2. Association according to claim 1, **characterised in that** 3-[(3-{[4-(4-morpholinylmethyl)-1*H*-pyrrol-2-yl]methylene}-2-oxo-2,3-dihydro-1*H*-indol-5-yl)methyl]-1,3-thiazolidine-2,4-dione is used in the form of the Z isomer.

3. Association according to either claim 1 or claim 2, **characterised in that** 3-[(3-{[4-(4-morpholinylmethyl)-1*H*-pyrrol-2-yl]methylene}-2-oxo-2,3-dihydro-1*H*-indol-5-yl)methyl]-1,3-thiazolidine-2,4-dione is used in the form of a hydrochloride.

4. Association according to either claim 1 or claim 2, **characterised in that** 3-[(3-{[4-(4-morpholinylmethyl)-1*H*-pyrrol-2-yl]methylene}-2-oxo-2,3-dihydro-1*H*-indol-5-yl)methyl]-1,3-thiazolidine-2,4-dione is used in the form of a mesylate.

5. Association according to any one of claims 1 to 4, **characterised in that** the EGFR tyrosine kinase inhibitor is gefitinib or erlotinib.

6. Association according to any one of claims 1 to 5 for use in the treatment of non-small-cell lung cancer.

7. Association according to any one of claims 1 to 5 for use in the treatment of non-small-cell lung cancer in patients who are resistant to an EGFR tyrosine kinase inhibitor.

8. Pharmaceutical composition comprising as active ingredient 3-[(3-{[4-(4-morpholinylmethyl)-1*H*-pyrrol-2-yl]methylene}-2-oxo-2,3-dihydro-1*H*-indol-5-yl)methyl]-1,3-thiazolidine-2,4-dione or a Z or E isomer thereof and/or an addition salt thereof with a pharmaceutically acceptable acid or base, in association with an EGFR tyrosine kinase inhibitor according to any one of claims 1 to 7 in combination with one or more pharmaceutically acceptable excipients.

9. Pharmaceutical composition according to claim 8 for use in the treatment of non-small-cell lung cancer.

10. Pharmaceutical composition according to claim 9 for use in the treatment of non-small-cell lung cancer in patients who are resistant to an EGFR tyrosine kinase inhibitor.
